# FASCICULE DE BREVET EUROPEEN

(11) **EP 1 317 299 B1**
(45) Date de publication et mention de la délivrance du brevet: **12.05.2004**
(21) Numéro de dépôt: 01967419.1
(22) Date de dépôt: 03.09.2001
(51) Int. Cl.: A61M 5/178, G21F 5/018

(54) **DISPOSITIF DE PROTECTION POUR SERINGUE, EN PARTICULIER POUR UNE SERINGUE SERVANT A L'INJECTION DE PRODUIT(S) RADIOACTIF(S)**
SCHUTZVORRICHTUNG FÜR EINE SPRITZE, INSBESONDERE FÜR EINE SPRITZE ZUM INJIZIEREN VON RADIOAKTIVER SUBSTANZEN
DEVICE FOR PROTECTING A SYRINGE, IN PARTICULAR FOR A SYRINGE USED FOR INJECTING RADIOACTIVE PRODUCT(S)

(30) Priorité: 01.09.2000 FR 0011370
(43) Date de publication de la demande: 11.06.2003
(73) Titulaire: Lemer Pax, 44477 Carquefou cedex (FR)
(72) Inventeur: LEMER, Pierre-Marie, F-44100 Nantes (FR)
(74) Mandataire: Catherine, Alain
(86) Numéro de dépôt international: PCT/FR2001/002728
(87) Numéro de publication internationale: WO 2002/017995

(56) Documents cités:
- EP-A- 0 661 066
- BE-A- 842 989
- FR-A- 2 308 382
- US-A- 4 185 619
- US-A- 4 615 468
- PATENT ABSTRACTS OF JAPAN vol. 1999, no. 09, 30 juillet 1999 (1999-07-30) & JP 11 114062 A (DAIICHI RADIOISOTOPE LABS LTD), 27 avril 1999 (1999-04-27)

## Description

La présente invention concerne les dispositifs du type « protège-seringue » qui équipent les seringues de type classique servant notamment à l'injection de produit(s) radioactif(s) pour assurer la protection des opérateurs contre l'exposition aux rayonnements de haute énergie (par exemple les rayonnements émis par l'iode 131, le fluor 18, l'oxygène 15 ou encore le carbone 11).

Les dispositifs de protection de ce genre qui sont actuellement sur le marché sont constitués d'une enveloppe de forme générale cylindrique en matériau radioprotecteur (plomb ; tungstène ...) adaptée pour recouvrir le corps cylindrique de la seringue. Cette enveloppe protectrice comporte un orifice au niveau de son extrémité avant qui permet le passage de l'aiguille d'injection ou du cône de réception de cette aiguille; elle comporte également un orifice au niveau de son extrémité arrière, qui est utilisé pour l'introduction et l'enlèvement du corps de seringue, ainsi que pour la manoeuvre de la tige du piston de seringue.

Ces structures remplissent relativement bien leur rôle protecteur mais il subsiste une possibilité d'irradiation par les orifices d'extrémités de l'enveloppe protectrice. En particulier, l'opérateur qui manoeuvre la seringue est directement soumis à ce risque d'irradiation par l'extrémité arrière de cette seringue, côté piston.

La présente invention propose une solution pour renforcer la protection procurée par ce genre de structure.

Le dispositif de protection pour seringue, conforme à la présente invention, comporte une enveloppe en matériau radioprotecteur, adaptée pour recouvrir le corps cylindrique de seringue, laquelle enveloppe est associée à une structure également en matériau radioprotecteur, qui est agencée pour former un bouclier protecteur au moins partiel du côté de l'extrémité arrière du corps de la seringue tout en autorisant la manoeuvre du piston de ladite seringue.

Toujours selon l'invention, l'enveloppe protectrice comporte une ouverture au niveau de son extrémité avant pour le passage de l'aiguille de la seringue ou du cône de réception de cette aiguille, et une ouverture au niveau de son extrémité arrière pour l'introduction et l'enlèvement de ladite seringue, ainsi que pour la manoeuvre de la tige de son piston ; en outre, il est prévu une structure rapportée en matériau radioprotecteur, qui est agencée pour former un bouclier protecteur au moins partiel en regard de l'ouverture aménagée au niveau de l'extrémité arrière de l'enveloppe protectrice, tout en autorisant la manoeuvre du piston de la seringue.

Selon un premier mode de réalisation, le bouclier protecteur se présente sous la forme générale d'une jaquette tubulaire munie d'un fond ; cette jaquette vient coiffer l'enveloppe protectrice par l'extrémité arrière de celle-ci et elle comporte des moyens de solidarisation amovibles avec la tige qui manoeuvre le piston de la seringue.

De préférence, la jaquette tubulaire en matériau radioprotecteur est montée coulissante sur l'enveloppe protectrice. D'autre part, le corps cylindrique de la jaquette tubulaire a avantageusement une longueur qui est adaptée pour venir coiffer l'extrémité arrière de l'enveloppe protectrice quel que soit le niveau d'extraction de la tige qui manoeuvre le piston de seringue.

Selon une autre caractéristique, les moyens de solidarisation amovibles de la jaquette tubulaire avec la tige qui manoeuvre le piston de seringue consistent en un verrou agencé pour plaquer la tête de ladite tige de manoeuvre contre la face interne du fond de ladite jaquette.

Selon une autre particularité, le dispositif de protection comporte des moyens qui permettent le verrouillage de la jaquette tubulaire sur l'enveloppe protectrice de manière à bloquer la position du piston de la seringue ; ces moyens particuliers sont avantageusement constitués de deux boutons moletés diamétralement opposés.
De préférence, ces boutons moletés sont solidaires de l'enveloppe protectrice et ils coopèrent avec des ouvertures longitudinales aménagées dans la jaquette tubulaire, lesquelles ouvertures débouchent au niveau de l'extrémité avant de cette jaquette.

Selon encore une autre particularité, le dispositif de protection conforme à la présente invention comporte des moyens de solidarisation amovibles du corps de seringue avec l'enveloppe protectrice. Ces moyens sont avantageusement constitués d'un verrou adapté pour plaquer la collerette d'extrémité du corps de seringue contre l'extrémité arrière de l'enveloppe protectrice ; l'organe de manoeuvre de ce verrou s'étend en saillie au travers d'une ouverture longitudinale aménagée dans la jaquette tubulaire, laquelle ouverture débouche au niveau de l'extrémité avant de ladite jaquette.

Selon un autre mode de réalisation, le bouclier protecteur est constitué de deux demi-pièces complémentaires escamotables agencées pour venir se positionner sur l'extrémité arrière de l'enveloppe protectrice (27), et dont les bordures de jointure sont conformées pour épouser le contour de la tige qui manoeuvre le piston de la seringue.

Les deux demi-pièces complémentaires peuvent être montées coulissantes sur un arceau de guidage positionné autour de l'extrémité arrière de l'enveloppe protectrice. Cet arceau assure le maintien des deux demi-pièces au niveau de l'extrémité arrière de l'enveloppe protectrice ; il permet le rapprochement des deux demi-pièces pour venir obturer l'ouverture de l'extrémité arrière de cette enveloppe protectrice autour de la tige de manoeuvre du piston de seringue, et il permet l'escamotage desdites demi-pièces de manière à autoriser le positionnement de la seringue dans l'enveloppe protectrice, ainsi que son enlèvement.

De préférence, les deux demi-pièces complémentaires sont munies d'épaulements inversés sur leurs bordures de jointure, constituant des moyens de recouvrement partiel. Elles comportent également avantageusement des prolongements qui forment une jupe de recouvrement de l'extrémité arrière de l'enveloppe protectrice.

Mais l'invention sera encore illustrée, sans être aucunement limitée, par la description suivante de plusieurs modes de réalisation particuliers, donnés uniquement à titre d'exemples et représentés sur les dessins annexés dans lesquels :
- la figure 1 est une vue de côté d'une première forme de réalisation possible d'un dispositif de protection de seringue conforme à la présente invention, la seringue protégée étant ici représentée avec sa tige de piston intégralement « rentrée » ;
- la figure 2 est une vue en coupe selon 2-2 de la figure 1 ;
- la figure 3 montre le même dispositif de protection avec la tige du piston de la seringue partiellement extraite ;
- la figure 4 est une vue en coupe selon 4-4 de la figure 3 ;
- la figure 5 est une vue en perspective d'une variante de réalisation du dispositif protège-seringue illustré sur les figures 1 à 4 ;
- la figure 6 est une vue en coupe longitudinale d'une autre forme de réalisation possible d'un dispositif de protection de seringue conforme à la présente invention, avec un bouclier protecteur, ici en position « ouverte », constitué de deux demi-pièces complémentaires ;
- la figure 7 est une vue en coupe selon 7-7 de la figure 6;
- la figure 8 est une vue similaire à la figure 7, le bouclier protecteur étant ici représenté en position « fermée » ;
- les figures 9 et 10 sont des vues en perspective, respectivement de ¾ arrière et de ¾ avant qui montrent le dispositif de protection des figures 6 à 8 avec le bouclier protecteur en position « ouverte » ;
- les figures 11 et 12 sont des vues en perspective, respectivement de ¾ avant et de ¾ arrière qui montrent le même dispositif de protection avec le bouclier protecteur en position « fermée ».

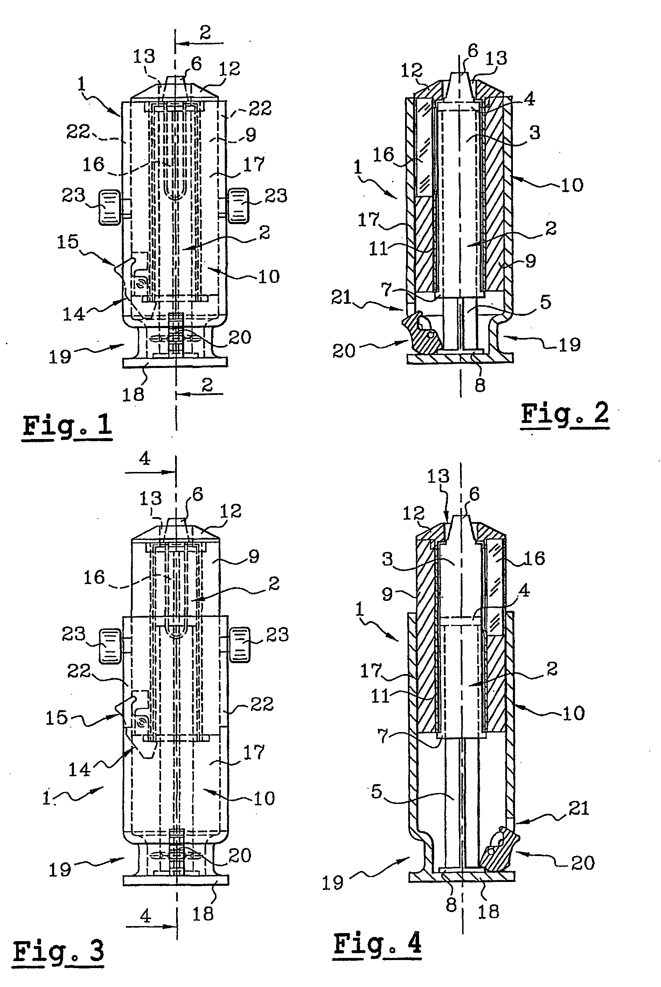

Les figures 1 à 4 illustrent une première forme de réalisation d'un dispositif 1 venant envelopper une seringue 2 pour protéger les opérateurs contre les rayonnements émis par le ou les produits radioactifs destinés à être injectés.

La seringue 2 est d'un type classique, constituée d'un corps de seringue cylindrique 3 associé à un piston interne 4 qui est manoeuvré par une tige 5 à section en croix. Le corps de seringue 3 se prolonge par un tronc de cône 6 au niveau de son extrémité avant, destiné à recevoir l'aiguille d'injection non représentée ; son extrémité arrière comporte une collerette périphérique 7.
D'autre part, l'extrémité externe de la tige 5 qui manoeuvre le piston 4 est équipée d'une tête plate 8.

Le dispositif protège-seringue 1 est principalement constitué d'une enveloppe cylindrique 9 qui entoure le corps de seringue 3, et d'une jaquette coulissante 10 qui constitue un bouclier de protection du côté de l'extrémité arrière de ladite enveloppe 9.

L'enveloppe cylindrique 9 est réalisée en matériau radioprotecteur, genre tungstène par exemple. Elle est doublée intérieurement par un fourreau 11 en matière plastique dont le diamètre interne correspond, au jeu près, au diamètre externe du corps de seringue 3. Ce fourreau 11 facilite le positionnement et le retrait du corps de seringue 3 au sein de l'enveloppe 9 ; selon le matériau utilisé, il peut également jouer un rôle dans la fonction de radioprotection.

L'extrémité avant de l'enveloppe protectrice 9 est équipée d'un capotage tronconique rapporté 12, également en tungstène, muni d'un orifice central 13 permettant le passage du tronc de cône 6 de la seringue. Cet orifice 13 est minimisé en dimension pour limiter la fuite des rayonnements ; le capotage 12 constitue également une butée d'extrémité pour le corps de seringue 3.

Au niveau de l'extrémité arrière de l'enveloppe cylindrique 9, on remarque la présence d'un moyen d'accrochagelrelargage rapide 14 qui permet de maintenir le corps de seringue 3 à l'intérieur de ladite enveloppe 9. Ce moyen d'accrochage/relargage 14 consiste en un verrou amovible qui vient plaquer la couronne d'extrémité 7 du corps de seringue 3 contre l'extrémité arrière de l'enveloppe 9 ; ce verrou est ici en forme de taquet coinceur manoeuvré manuellement par l'intermédiaire d'une extension 15 en saillie, et soumis à un ressort de rappel.

Du côté de l'extrémité avant de l'enveloppe cylindrique 9, on remarque la présence d'un hublot 16 en verre au plomb qui permet d'accéder visuellement à la partie utile des graduations du corps de seringue 3.

La jaquette 10 qui est associée à l'enveloppe protectrice 9 est également réalisée en matériau radioprotecteur, genre tungstène par exemple. Cette jaquette 10 se présente sous la forme générale d'un tube cylindrique 17 dont le diamètre interne correspond, au jeu près, au diamètre externe de l'enveloppe 9, et dont l'une des extrémités est obturée par un élément de fond 18. Juste devant l'élément de fond 18 on remarque la présence d'un creux annulaire 19 aménagé dans la partie tubulaire 17.

Au niveau de la face interne de l'élément de fond 18, un verrou amovible 20 en forme de taquet coinceur est agencé pour permettre l'accrochage de la tige 5 qui manoeuvre le piston de seringue 4. Ce verrou 20 a une structure similaire à celle de l'organe de verrouillage 14 décrit précédemment; il est accessible par une ouverture 21 aménagée dans la partie tubulaire 17, au niveau du creux annulaire 19.

Sur une partie de la longueur de l'élément tubulaire 17 on a prévu deux ouvertures longitudinales 22 diamétralement opposées visibles sur les figures 1 et 3. Ces deux ouvertures 22 débouchent au niveau de l'extrémité avant de l'élément tubulaire 17 ; elles sont destinées à coopérer avec des boutons moletés 23 solidaires de l'enveloppe cylindrique 9 pour permettre un verrouillage de la jaquette tubulaire 10 sur ladite enveloppe 9. L'une des deux ouvertures longitudinales 22 sert également au passage de l'extension 15 du taquet coinceur 14.

La jaquette 10 est mise en place sur l'enveloppe cylindrique 9 par l'extrémité arrière de celle-ci, après que la seringue 2 ait été introduite dans sa structure d'accueil 9, 11. Le tube cylindrique 17 est guidé sur l'enveloppe 9 jusqu'à ce que la tête plate 8 de la tige de manoeuvre 5 vienne se solidariser automatiquement avec l'élément de fond 18 de la jaquette, sous l'action du verrou 20. Dans cette position, la jaquette 10 forme un bouclier qui protège l'opérateur des rayonnements émis dans l'axe de la seringue par l'ouverture arrière de l'enveloppe protectrice 9.

La seringue 2 est alors prête à être utilisée, la manoeuvre de son piston 4 étant réalisée par coulissement de la jaquette 10 sur l'enveloppe 9. Ce coulissement est possible lorsque les boutons moletés 23 sont desserrés ; ces boutons moletés 23 ainsi que la réservation annulaire 19 peuvent alors être utilisés à titre de points d'appui pour les doigts, lors du remplissage de la seringue ou lors de l'injection du produit.
Les boutons moletés 23 sont serrés lorsque l'on veut verrouiller le positionnement de la jaquette 10 sur l'enveloppe protectrice 9 ; ce verrouillage permet de bloquer la position du piston 4 dans le corps de seringue 3 et on évite ainsi tout écoulement accidentel du produit radioactif par l'aiguille d'injection.

La partie tubulaire 17 de la jaquette 10 a une longueur qui est adaptée pour venir coiffer l'extrémité arrière de l'enveloppe protectrice 9 quel que soit le niveau d'extraction de la tige 5. En fonction de ce niveau d'extraction, cette partie tubulaire 17 vient doubler plus ou moins l'enveloppe cylindrique 9 et elle renforce donc la protection conférée par cette dernière.

Tel que représenté sur les figures 1 et 2, la longueur de la partie tubulaire 17 est adaptée de sorte à venir recouvrir complètement l'enveloppe 9 lorsque le piston 4 de la seringue est intégralement rentré. Les figures 3 et 4 montrent la seringue avec la tige 5 qui manoeuvre. le piston 4 partiellement extraite.

Lorsque l'injection du produit radioactif est terminée, la seringue est extraite de son dispositif protecteur par une manoeuvre des deux taquets coinceurs 14 et 20.

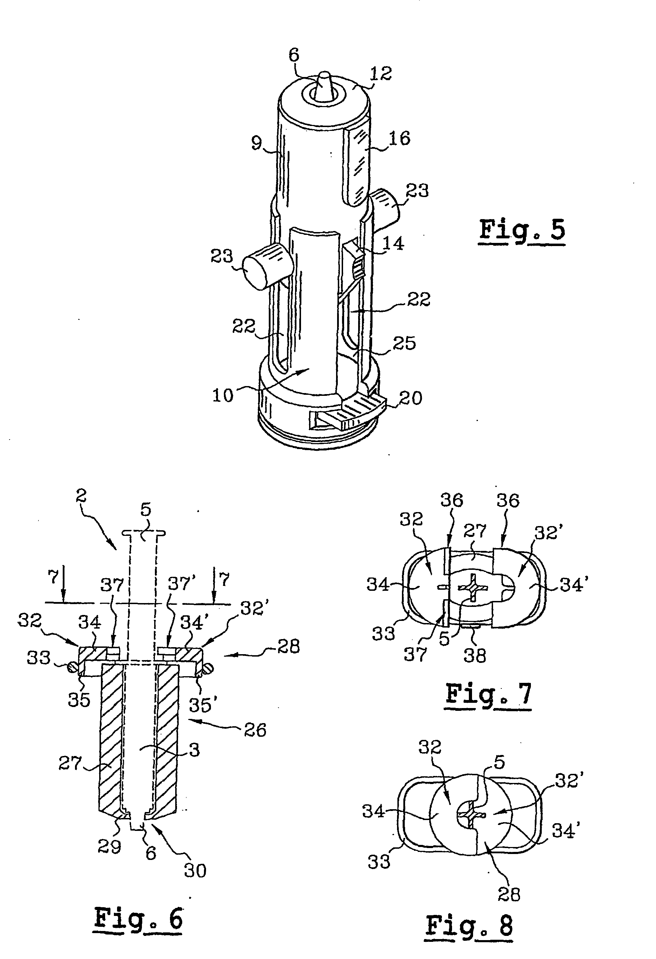

On a représenté sur la figure 5 une variante de réalisation du dispositif de protection de seringue illustré sur les figures 1 à 4.
On retrouve ici l'enveloppe protectrice 9 avec son hublot blindé 16 et avec son capotage 12 par l'orifice central duquel passe le tronc de cône d'extrémité 6 de la seringue. On retrouve encore la jaquette tubulaire 10 avec ses rainures longitudinales débouchantes 22 qui coopèrent avec les boutons moletés 23 solidaires de l'enveloppe 9.
Dans cette variante de réalisation, la partie d'extrémité arrière de la jaquette 10 a été légèrement modifiée ; elle ne comporte pas de creux annulaire et le verrou amovible 20 correspond à un modèle différent de celui illustré sur les figures 1 à 4.
D'autre part, le verrou 14 qui assure une solidarisation amovible entre le corps de seringue et l'enveloppe protectrice 9 s'étend au travers d'une rainure longitudinale 25 qui lui est propre, indépendante des deux rainures 22 servant au passage du pied des boutons moletés 23.

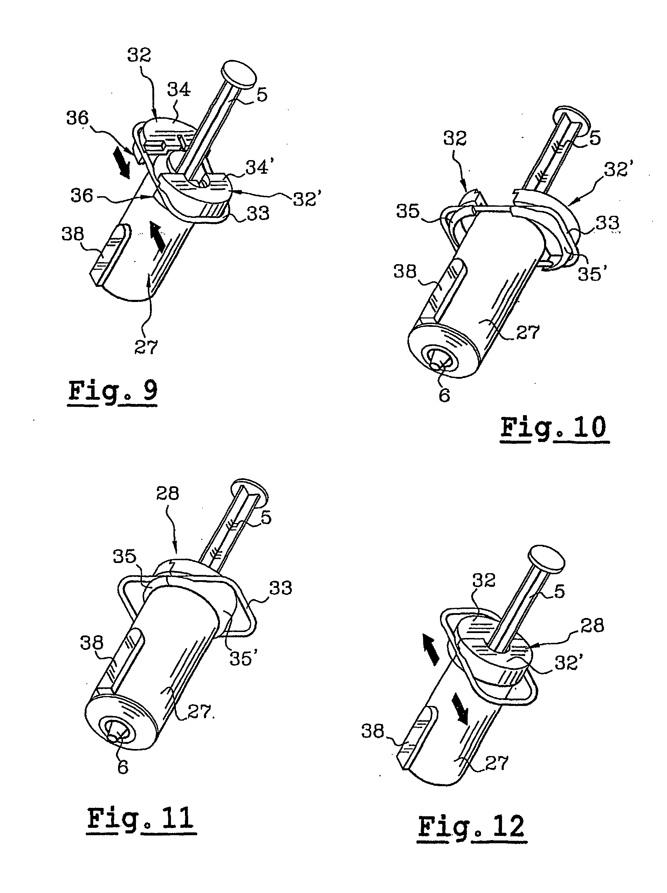

Les figures 6 à 12 illustrent un autre mode de réalisation possible d'un dispositif protège-seringue conforme à la présente invention. Dans la description qui suit on a conservé les repères précédents affectés à la seringue d'injection pour des motifs de simplification.

Ce dispositif protège-seringue 26 est constitué d'une enveloppe cylindrique 27 en matériau radioprotecteur genre tungstène, associée à un bouclier rapporté 28, également en matériau radioprotecteur genre tungstène, permettant une obturation amovible de l'ouverture arrière de ladite enveloppe 27.

L'enveloppe cylindrique 27 reçoit le corps de seringue 3 ; son extrémité avant comporte un retour annulaire intérieur 29 qui ne laisse subsister qu'un petit orifice central 30 juste suffisant pour le passage du tronc de cône d'extrémité 6 de la seringue.

Le bouclier rapporté 28 se compose de deux demi-pièces complémentaires 32 et 32', ici en forme de demi-coquilles, qui sont adaptées pour venir se positionner sur l'extrémité arrière de l'enveloppe cylindrique 27. Ces deux demi-coquilles 32 et 32' sont montées coulissantes sur un arceau support 33 qui, tout en assurant leur solidarisation, autorise leur rapprochement et leur éloignement dans un plan perpendiculaire à l'axe de l'enveloppe protectrice 27. Les deux portions parallèles rectilignes de l'arceau 36, qui constituent les portions de guidage des deux demi-coquilles 32 et 32', sont légèrement serrées contre l'enveloppe cylindrique 27 pour assurer le maintien en position du bouclier 28. Pour sécuriser ce maintien, on peut aussi prévoir d'encastrer la partie centrale de ces portions rectilignes dans des gorges conformées sur la périphérie de l'enveloppe protectrice 9.

Lorsque les deux demi-coquilles 32 et 32' sont écartées l'une de l'autre, le corps de seringue 3 peut être introduit dans l'enveloppe protectrice 27 (figures 6, 7, 9 et 10) ; une fois la seringue en place, ces deux demi-coquilles sont rapprochées manuellement pour venir obturer l'orifice arrière de ladite enveloppe 27 tout en enserrant la tige 5 qui manoeuvre le piston de la seringue (figures 8, 11 et 12).

Les deux demi-coquilles 32 et 32' sont constituées d'un élément d'obturation 34, 34' de forme générale demi-circulaire, prolongé par une portion de jupe demi-cylindrique 35, 35'. Ces portions de jupe 35, 35' viennent enserrer l'extrémité arrière de l'enveloppe 27 lorsque les deux demi-coquilles 32 et 32' , sont placées en position active de radioprotection ; elles comportent des rainures latérales 36 qui assurent le guidage des demi-coquilles 32, 32' sur les tronçons rectilignes parallèles de l'arceau 33.

Les bordures de jointure des deux éléments d'obturation 34 et 34' sont conformées pour épouser au mieux le contour de la tige 5 à section en croix, lorsqu'ils sont rapprochés l'un de l'autre. Seule alors la surface correspondant à la section en croix de cette tige 5 n'est pas protégée, ce qui limite les possibilités de fuite de rayonnements. D'autre part, ces bordures de jointure comportent également des épaulements inversés, 37, 37'. qui assurent un recouvrement partiel des deux éléments d'obturation 37, 37' lorsqu'ils sont juxtaposés, ce qui permet de sécuriser la radioprotection.

Une fois les deux demi-coquilles 32, 32' rapprochées l'une de l'autre, la tige 5 de la seringue peut être manoeuvrée de manière classique par coulissement au travers du bouclier protecteur 28.

Sur les figures 7 et 9 à 12, on remarque la présence d'un hublot blindé 38, en verre au plomb, qui équipe l'extrémité avant de l'enveloppe cylindrique 27 pour accéder visuellement aux graduations du corps de seringue 3.

Selon encore une autre forme de réalisation possible l'enveloppe cylindrique qui entoure le corps de seringue 3 peut être associée à une pastille par exemple circulaire, réalisée en matériau radioprotecteur, fixée de manière amovible sur la tête plate 8 du piston de seringué 4.

## Revendications

1. Dispositif de protection pour une seringue (2) de type classique, en particulier pour une seringue servant à l'injection de produit(s) radioactif(s), lequel dispositif comprend une enveloppe (9, 27) en matériau radioprotecteur, adaptée pour recouvrir le corps cylindrique (3) de la seringue, laquelle enveloppe (9, 27) est munie d'une ouverture au niveau de son extrémité avant pour le passage de l'aiguille de la seringue (2) ou du cône (6) de réception de cette aiguille, et d'une ouverture au niveau de son extrémité arrière pour l'introduction et l'enlèvement de ladite seringue (2), ainsi que pour la manoeuvre de la tige (5) de son piston (4), **caractérisé en ce que** ladite enveloppe (9, 27) est associée à une structure (10, 28), également en matériau radioprotecteur, laquelle structure (10, 28) est agencée pour former un bouclier protecteur au moins partiel en regard de l'ouverture aménagée au niveau de l'extrémité arrière de l'enveloppe protectrice (9, 27), tout en autorisant la manoeuvre du piston (4) de la seringue (2).

2. Dispositif selon la revendication 1, **caractérisé en ce qu'**il comporte un bouclier protecteur qui se présente sous la forme générale d'une jaquette tubulaire (10) munie d'un fond (18), laquelle jaquette (10) vient coiffer l'enveloppe protectrice (9) par l'extrémité arrière de celle-ci, et laquelle jaquette (10) comporte des moyens (20) de solidarisation amovibles avec la tige (5) qui manoeuvre le piston (4) de la seringue.

3. Dispositif selon la revendication 2, **caractérisé en ce qu'**il comporte une jaquette tubulaire (10) montée coulissante sur l'enveloppe protectrice (9).

4. Dispositif selon la revendication 3, **caractérisé en ce que** le corps cylindrique (17) de la jaquette tubulaire (10) a une longueur qui est adaptée pour venir coiffer l'extrémité arrière de l'enveloppe protectrice (9) quel que soit le niveau d'extraction de la tige (5) qui manoeuvre le piston (4) de la seringue.

5. Dispositif selon l'une quelconque des revendications 2 à 4, **caractérisé en ce que** les moyens de solidarisation amovibles de la jaquette tubulaire (10) avec la tige (5) qui manoeuvre le piston (4) de la seringue consistent en un verrou (20) agencé pour plaquer la tête (8) de ladite tige de manoeuvre (5) contre la face interne du fond (18) de ladite jaquette (10).

6. Dispositif selon l'une quelconque des revendications 2 à 5, **caractérisé en ce qu'**il comporte des moyens (22, 23) qui permettent de verrouiller le positionnement de la jaquette tubulaire (10) sur l'enveloppe protectrice (9).

7. Dispositif selon la revendication 6, **caractérisé en ce que** les moyens qui permettent le verrouillage de la jaquette tubulaire (10) sur l'enveloppe protectrice (9) sont constitués de deux boutons moletés (23) diamétralement opposés.

8. Dispositif selon la revendication 7, **caractérisé en ce qu'**il comporte des boutons moletés (23) solidaires de l'enveloppe protectrice (9) qui coopèrent avec des ouvertures longitudinales (22) aménagées dans la jaquette tubulaire (10), lesquelles ouvertures (22) débouchent au niveau de l'extrémité avant de ladite jaquette (10).

9. Dispositif selon la revendication 8, **caractérisé en ce qu'**il comporte des moyens (14) de solidarisation amovibles du corps de seringue (3) avec l'enveloppe protectrice (9), lesquels moyens sont constitués d'un verrou (14) adapté pour plaquer la collerette d'extrémité (7) du corps de seringue (3) contre l'extrémité arrière de ladite enveloppe protectrice (9), l'organe de manoeuvre (15) dudit verrou (14) s'étendant en saillie au travers d'une ouverture longitudinale (22, 25) aménagée dans la jaquette tubulaire (10), laquelle ouverture (22, 25) débouche au niveau de l'extrémité avant de ladite jaquette (10).

10. Dispositif selon la revendication 1, **caractérisé en ce qu'**il comporte un bouclier protecteur (28) constitué de deux demi-pièces complémentaires escamotables (32, 32') agencées pour venir se positionner sur l'extrémité arrière de l'enveloppe protectrice (27) et dont les bordures de jointure sont conformées pour épouser le contour de la tige (5) qui manoeuvre le piston (4) de la seringue.

11. Dispositif selon la revendication 10, **caractérisé en ce qu'**il comporte un bouclier protecteur (28) constitué de deux demi-pièces complémentaires (32, 32') montées coulissantes sur un arceau de guidage (33) positionné autour de l'extrémité arrière de l'enveloppe protectrice (27).

12. Dispositif selon l'une quelconque des revendications 10 ou 11, **caractérisé en ce qu'**il comporte un bouclier protecteur (28) constitué de deux demi-pièces complémentaires (32, 32') munies d'épaulements inversés (37, 37') sur leurs bordures de jointure, constituant des moyens de recouvrement partiel.

13. Dispositif selon l'une quelconque des revendications 10 à 12, **caractérisé en ce que** les deux demi-pièces complémentaires (32, 32') qui constituent le bouclier protecteur (28) comportent des prolongements (35, 35') qui forment une jupe de recouvrement de l'extrémité arrière de l'enveloppe protectrice (27).

14. Dispositif selon la revendication 1, **caractérisé en ce qu'**il comporte un bouclier protecteur en forme de pastille, par exemple circulaire, réalisée en matériau radioprotecteur, destiné à venir se fixer de manière amovible sur la tête plate (8) du piston de seringue (4).

## Claims

1. A protection device for a syringe (2) of conventional type, in particular for a syringe used for the injection of radioactive product(s), which device comprises a covering (9, 27) of radioprotective material, suited to cover the cylindrical body (3) of the syringe, which covering (9, 27) is provided with an opening at its front end for letting through the needle of the syringe (2) or the reception cone (6) of this needle, and with an opening at its rear end for the insertion and the withdrawal of said syringe (2), as well as for operating of the stem (5) of its piston (4), **characterised in that** said covering (9, 27) is associated with a structure (10, 28), also of radioprotective material, which structure (10, 28) is suitable to form a protective shield at least partially facing the opening provided at the rear end of the protection covering (9, 27) while enabling the operation of the piston (4) of said syringe (2).

2. A device according to claim 1, **characterised in that** it comprises a protective shield which has the general form of a tubular jacket (10) provided with a bottom (18), which jacket (10) covers the protective covering (9) by the rear end thereof, and which jacket (10) comprises removable interconnection means (20) with the stem (5) which operates the piston (4) of the syringe.

3. A device according to claim 2, **characterised in that** it comprises a tubular jacket (10) mounted to slide over the protective covering (9).

4. A device according to claim 3, **characterised in that** the cylindrical body (17) of the tubular jacket (10) has a length which is suited to cover the rear end of the protective covering (9) regardless of the extraction level of the stem (5) which operates the piston (4) of the syringe.

5. A device according to any one of claims 2 to 4, **characterised in that** the removable interconnection means of the tubular jacket (10) with the stem (5) which operates the piston (4) of the syringe comprise of a lock (20) arranged to press the head (8) of said operating stem (5) against the internal face of the bottom (18) of said jacket (10).

6. A device according to any one of claims 2 to 5, **characterised in that** it comprises means (22, 23) which enable to lock the positioning of the tubular jacket (10) to the protective covering (9).

7. A device according to claim 6, **characterised in that** the means which enable to lock the tubular jacket (10) to the protective covering (9) comprise two diametrically opposed knurled knobs (23).

8. A device according to claim 7, **characterised in that** it comprises knurled knobs (23) integral with the protective covering (9) which co-operate with longitudinal openings (22) arranged in the tubular jacket (10), which openings (22) emerge at the front end of said jacket (10).

9. A device according to claim 8, **characterised in that** it comprises removable interconnection means (14) of the syringe body (3) with the protective covering (9), which means comprise a lock (14) suited to press the end collar (7) of the syringe body (3) against the rear end of said protective covering (9), whereas the operating member(15) of said lock (14) protrudes across a longitudinal opening (22, 25) arranged in the tubular jacket (10), which opening (22, 25) emerges at the front end of said jacket (10).

10. A device according to claim 1, **characterised in that** it comprises a protective shield (28) consisting of two additional retractable half-parts (32, 32') provided to be positioned on the rear end of the protective covering (27) and whereof the junction edges are formed to match the contour of the stem (5) which operates the piston (4) of the syringe.

11. A device according to claim 10, **characterised in that** it comprises a protective shield (28) consisting of two additional half-parts (32, 32') mounted to slide in a guiding cradle (33) positioned around the rear end of the protective envelope (27).

12. A device according to any one of claims 10 or 11, **characterised in that** it comprises a protective shield (28) consisting of two additional half-parts (32, 32') provided with reverse shoulders (37, 37') on their junction edges, forming partial covering means.

13. A device according to any one of claims 10 to 12, **characterised in that** both additional half-parts (32, 32') which constitute the protective shield (28) comprise extensions (35, 35') which form a covering skirt of the rear end of the protective covering (27).

14. A device according to claim 1, **characterised in that** it comprises a protective shield which has the general form of a pellet, for example circular, made of radio-protective material, provided to be removably fixed on the flat head (8) of the syringe piston (4).

## Patentansprüche

1. Schutzvorrichtung für eine Spritze (2) herkömmlichen Typs, insbesondere für eine Spritze zum Einspritzen von radioaktiven Stoffen, wobei die Vorrichtung eine Hülle (9, 27) aus einem Strahlenschutzmaterial umfasst, die derart ausgeführt ist, dass sie den zylindrischen Körper (3) der Spritze aufnimmt, wobei die Hülle (9, 27) mit einer Öffnung auf Höhe ihres vorderen Endes für den Durchgang der Nadel der Spritze (2) oder des Aufnahmekegels (6) für diese Nadel und mit einer Öffnung auf Höhe ihres hinteren Endes für die Einführung und Entnahme dieser Spritze (2) sowie für die Betätigung der Stange (5) ihres Kolbens (4) versehen ist, **dadurch gekennzeichnet, dass** die Hülle (9, 27) mit einem Aufbau (10, 28) ebenfalls aus einem Strahlenschutzmaterial verbunden ist, wobei der Aufbau (10, 28) derart angeordnet ist, dass er einen zumindest teilweisen Schutzschild gegenüber der Öffnung bildet, die auf Höhe des hinteren Endes der Schutzhülle (9, 27) angeordnet ist, und gleichzeitig die Betätigung des Kolbens (4) der Spritze (2) gestattet.

2. Vorrichtung nach Anspruch 1, **dadurch gekennzeichnet, dass** sie einen Schutzschild umfasst, der in der allgemeinen Form eines röhrenförmigen Umschlags (10) vorgesehen ist, der mit einem Boden (18) versehen ist, wobei der Umschlag (10) an der Schutzhülle (9) an dem hinteren Ende derselben anliegt, und wobei dieser Umschlag abnehmbare Mittel (20) zur Befestigung mit der Stange (5) umfasst, die den Kolben (4) der Spritze betätigt.

3. Vorrichtung nach Anspruch 2, **dadurch gekennzeichnet, dass** sie einen röhrenförmigen Umschlag (10) umfasst, der gleitend auf der Schutzhülle (9) befestigt ist.

4. Vorrichtung nach Anspruch 3, **dadurch gekennzeichnet, dass** der zylindrische Körper (17) des röhrenförmigen Umschlags (10) eine Länge aufweist, die derart angepasst ist, dass sie an dem hinteren Ende der Schutzhülle (9) anliegt, unabhängig von der Ausziehhöhe der Stange (5), die den Kolben (4) der Spritze betätigt.

5. Vorrichtung nach einem der Ansprüche 2 bis 4, **dadurch gekennzeichnet, dass** die verstellbaren Befestigungsmittel des röhrenförmigen Umschlags (10) an der Stange (5), die den Kolben (4) der Spritze betätigt, aus einem Riegel (20) bestehen, der derart angeordnet ist, dass er den Kopf (8) der Betätigungsstange (5) an der Innenseite des Bodens (18) des Umschlags (10) anlegt.

6. Vorrichtung nach einem der Ansprüche 2 bis 5, **dadurch gekennzeichnet, dass** sie Mittel (22, 23) umfasst, die es ermöglichen, die Position des röhrenförmigen Umschlags (10) auf der Schutzhülle (9) zu verriegeln.

7. Vorrichtung nach Anspruch 6, **dadurch gekennzeichnet, dass** die Mittel, die die Verriegelung des röhrenförmigen Umschlags (10) auf der Schutzhülle (9) ermöglichen, aus zwei diametral gegenüber liegenden Rändelrädern (23) gebildet sind.

8. Vorrichtung nach Anspruch 7, **dadurch gekennzeichnet, dass** sie Röndelräder (23) umfasst, die mit der Schutzhülle (9) verbunden sind und mit länglichen Öffnungen (22) zusammenwirken, die in dem röhrenförmigen Umschlag (10) vorgesehen sind, wobei diese Öffnungen (22) auf Höhe des vorderen Endes des Umschlags (10) münden.

9. Vorrichtung nach Anspruch 8, **dadurch gekennzeichnet, dass** sie verstellbare Befestigungsmittel (14) des Spritzenkörpers (3) an der Schutzhülle (9) umfasst, wobei die Mittel aus einem Riegel (14) gebildet sind, der derart ausgeführt ist, dass er den Endkragen (7) des Spritzenkörpers (3) an dem hinteren Ende der Schutzhülle (9) anlegt, wobei sich das Betätigungselement (15) dieses Riegels (14) vorspringend durch eine längliche Öffnung (22, 25) erstreckt, die in dem röhrenförmigen Umschlag (10) vorgesehen ist, wobei die Öffnung (22, 25) auf Höhe des vorderen Endes des Umschlags (10) mündet.

10. Vorrichtung nach Anspruch 1, **dadurch gekennzeichnet, dass** sie einen Schutzschild (28) umfasst, der aus zwei einziehbaren komplementären halben Teilen (32, 32') gebildet ist, die derart angeordnet sind, dass sie sich am hinteren Ende der Schutzhülle (27) positionieren, und dessen Verbindungsränder derart ausgebildet sind, dass sie sich an die Kontur der Stange (5), die den Kolben (4) der Spritze betätigt, anlegen.

11. Vorrichtung nach Anspruch 10, **dadurch gekennzeichnet, dass** sie einen Schutzschild (28) umfasst, der aus zwei komplementären halben Teilen (32, 32') gebildet ist, die gleitend auf einem Führungsbogen (33) befestigt sind, der um das hintere Ende der Schutzhülle (27) herum angeordnet ist.

12. Vorrichtung nach einem der Ansprüche 10 oder 11, **dadurch gekennzeichnet, dass** sie einen Schutzschild (28) umfasst, der aus zwei komplementären halben Teilen (32, 32') gebildet ist, die mit umgekehrten Ansätzen (37, 37') an ihren Verbindungsrändern versehen sind, die Teilabdeckungsmittel bilden.

13. Vorrichtung nach einem der Ansprüche 10 bis 12, **dadurch gekennzeichnet, dass** die beiden komplementären halben Teile (32, 32'), die den Schutzschild (28) bilden, Verlängerungen (35, 35') umfassen, die eine Schürze zur Abdeckung des hinteren Endes der Schutzhülle (27) bilden.

14. Vorrichtung nach Anspruch 1, **dadurch gekennzeichnet, dass** sie einen Schutzschild in Form einer beispielsweise kreisförmigen Tablette umfasst, die aus einem Strahlenschutzmaterial hergestellt und dazu bestimmt ist, abnehmbar auf dem flachen Kopf (8) des Spritzenkolbens (4) befestigt zu werden.
